# EUROPÄISCHE PATENTSCHRIFT

(11) **EP 0 412 571 B1**
(45) Veröffentlichungstag und Bekanntmachung des Hinweises auf die Patenterteilung: **23.11.1994**
(21) Anmeldenummer: 90115428.6
(22) Anmeldetag: 10.08.1990
(51) Int. Cl.: A61L 2/26, E05B 65/10

(54) **Sicherungssiegel für Sterilisierbehälter**
Safety seal for sterilisation containers
Soupape de sûreté pour un récipient pour stérilisation

(30) Priorität: 10.08.1989 DE 3926523
(43) Veröffentlichungstag der Anmeldung: 13.02.1991
(73) Patentinhaber: Wagner GmbH Fabrik für medizinische Geräte, 80634 München (DE)
(72) Erfinder: Wagner, Peter, D-8130 Starnberg (DE)
(74) Vertreter: Koch, Günther, Dipl.-Ing.

(56) Entgegenhaltungen:
- WO-A-79/00077
- WO-A-81/02108
- WO-A-87/07151
- DE-A- 3 710 049
- FR-A- 2 335 239

## Beschreibung

Die Erfindung betrifft ein Sicherungssiegel für Sterilisierbehälter mit einer definierten Schließstellung, welche gewährleistet, daß der Sterilisierbehälter seit seiner Sterilisation geschlossen gehalten wurde und mit einer Öffnungsstellung, die auf eine zwischenzeitliche Öffnung hinweist.

Derartige Sicherungssiegel werden für Sterilisierbehälter benutzt, in denen chirurgische Instrumente oder anderes Sterilgut während des Sterilisiervorganges und danach bis zur Bereitstellung verbleiben. Der unverletzte Zustand eines solchen Sicherungssiegels soll dem Benutzer erkennbar machen, ob der Deckel seit Durchführung des Sterilisiervorganges bereits wieder vom Behälterunterteil abgenommen wurde oder auf diesem Behälter ungeöffnet verblieb, woraus geschlossen werden kann, ob der Inhalt durch Keime verunreinigt sein kann oder nicht.

Die bisher bekannten Sicherungssiegel in Gestalt vom Plomben bestehen aus Kunststoffverschlußringen, die an den Behälterverschlüssen so angebracht werden müssen, daß der Verschluß nur durch irreversiblen Bruch der Plombe geöffnet werden kann. Diese nur einmal benutzten Plomben aus Kunststoff stellen einen zusätzlichen Kostenfaktor dar.

Es ist ferner bekannt, an Sterilisierbehältern Indikatoren anzubringen, die auf die während des Sterilisiervorganges auftretende Wärme (oder eine vorbestimmte Druckdifferenz) ansprechen und anzeigen, daß der betreffende Behälter den Sterilisierzyklus durchlaufen hat.

Ein solches Sicherungssiegel ist nicht notwendig für Sterilisierbehälter, die während des Sterilisiervorganges in der abschließenden Vakuumphase dichtend abgeschlossen und nach Entnahme aus dem Sterilisator durch den äußeren Luftdruck geschlossen gehalten werden. Bei derartigen Sterilisierbehältern wird dem Benutzer ein vorheriges Öffnen des Deckels bereits dadurch erkennbar, daß das Vakuum fehlt und der Deckel ohne Betätigung eines Ventils abgenommen werden kann. Derartige Sterilisierbehälter, die unter Vakuum verschließbar sind, zeigen die WO-A-79/00077, die WO-A-81/02108 und die FR-A-2 335 239. All diesen bekannten Sterilisierbehältern ist eine Anordnung gemeinsam, die den Sterilisierbehälter während des Sterilisiervorganges geöffnet hält, d.h. das Innere des Sterilisierbehälters mit der Atmosphäre des Sterilisators verbindet und am Ende des Sterilisiervorganges in der Vakuumphase den Sterilisierbehälter vakuumdicht schließt.

Gemäß der WO-A-79/00077 geschieht dies durch ein Ventil, welches einen auf einen Ventilkörper einwirkenden Balg aufweist, der zunächst in der Vorvakuumphase mit der Atmosphäre des Sterilisators in Verbindung steht, dann durch einen Schrumpfschlauch geschlossen wird und in der abschließenden Vakuumphase beim Aufblähen das Ventil schließt.

Eine ähnliche Anordnung hält bei der WO-A-81/02108 den Behälterdeckel geöffnet, wobei in der abschließenden Vakuumphase die Deckelhalterung weggezogen wird, so daß der Deckel auf dem Behälterunterteil zu liegen kommt und vakuumdicht geschlossen wird.

Die in der FR-A-2 335 239 beschriebene Ventilanordnung benutzt ein Eutektikum, welches bei Erreichen einer vorbestimmten Temperatur ein Ventilverschlußglied freigibt.

Der vorliegenden Erfindung liegt demgegenüber die Aufgabe zugrunde, ein Sicherungssiegel für Sterilisierbehälter gemäß dem Oberbegriff der Patentansprüche 1 und 2 so auszubilden, daß es bei einfacher Handhabung wiederverwendbar ist und zusätzlich als Sterilisationsprozeßindikator verwendet werden kann.

Gelöst wird die gestellte Aufgabe durch eine Temperatursteuerung gemäß dem Kennzeichnungsteil des Patentanspruchs 1 oder eine Drucksteuerung gemäß dem Kennzeichnungsteil des Patentanspruchs 2.

Das Wesen der Erfindung besteht demgemäß darin, daß die Plombe reversibel wieder geöffnet werden kann, aber nur durch den Sterilisationsvorgang (Temperatur oder/und Druck) in die Schließstellung überführt werden kann. Damit unterscheidet sich das erfindungsgemäße Sicherungssiegel grundsätzlich von den bekannten Plomben, die durch manuelle Handhabung verschlossen werden mußten und dann nur noch durch Bruch lösbar waren.

Dieses Sicherungssiegel-Prinzip kann durch eine Überholkupplung, d.h. eine in einer Richtung als Freilauf ausgebildete Kupplung verwirklicht werden, wobei der Freilauf während der Öffnungsbewegung wirksam wird und in Gegenrichtung eine Einkupplung stattfindet, mit der eine Bewegung erst dann möglich ist, wenn ein Thermofühler oder ein Druckfühler während des Sterilisiervorganges den Bewegungsablauf freigeben. Der Thermosensor ist dabei zweckmäßigerweise als Legierung, insbesondere als eutektische Legierung ausgebildet, die bei einer vorbestimmbaren Temperatur schmilzt.

Gemäß einem ersten Ausführungsbeispiel kann das Sicherungssiegel als vom Sterilisierbehälter getrenntes Einzelteil in Gestalt eines Vorhängeschlosses ausgebildet sein, welches ähnlich wie die herkömmlichen Kunststoffplomben an beliebigen Behälterverschlüssen anbringbar sind. Das erfindungsgemäße Sicherungssiegel wird dann im geöffneten Zustand an den Behälterverschluß gehängt und im Gegensatz zu den herkömmlichen Plomben nicht mechanisch verschlossen. Durch die darauf folgende Sterilisation und nur durch diese wird das Sicherungssiegel während des Sterilisiervorganges, vorzugsweise bei Erreichen der Sterilisiertemperatur geschlossen, womit der Behälter versiegelt ist. Soll der Verschluß geöffnet werden, muß das Sicherungssiegel in die Öffnungsstellung überführt werden, was über den Freilauf der Kupplung erfolgen kann. Hat das Siegel dann die Öffnungsstellung erreicht, kann es manuell ohne mutwillige Zerstörung nicht mehr geschlossen werden, sondern nur durch erneute Sterilisation.

Die wesentlichen Vorteile der Erfindung bestehen demgemäß darin, daß ein wiederverwendbares und damit billiges, abfallfreies Siegel geschaffen wird. Zusätzlich wirkt z.B. eine Ausführung mit Eutektikum gleichzeitig als Prozeßindikator, da das Siegel nur dann in die Schließstellung überführt werden kann, wenn die Sterilisiertemperatur erreicht worden ist, wohingegen herkömmliche Plomben nicht beweisen können, daß sterilisiert wurde, und daher einen zusätzlichen Indikator benötigen. Ausführungen mit Bimetall-, Flüssigkeits- oder Gasausdehnung beweisen zwar auch, daß der Sterilisierbehälter erwärmt (sterilisiert) wurde, können aber im Gegensatz zur vorzuziehenden Lösung mit Eutektikum nicht beweisen, daß die Sterilisiertemperatur auch genau erreicht wurde (Eutektikum auf 0,2° C).
Gemäß einer weiteren Ausgestaltung der Erfindung kann das Sicherungssiegel in den Behälterverschluß eingebaut werden und dort die beschriebene Funktion ausüben.

Nach einer weiteren zweckmäßigen Ausgestaltung kann einem solchen im Behälterverschluß eingebauten Sicherungssiegel noch eine weitere Funktion auferlegt werden. Danach kann das Siegel so ausgebildet sein, daß beim Übergang in den Schließzustand während des Sterilisiervorganges ein Anpressen des Deckels auf das Behälterunterteil erfolgt, so daß die dazwischen eingelegte Dichtung unter Druck gesetzt wird und zuverlässig schließt. Zu diesem Zweck kann eine thermische Ausdehnung oder Ausbiegung Anwendung finden, oder es wird die durch Druckabsenkung benutzte Verschiebung eines Stellgliedes benutzt, um einerseits den Verschluß in Spannstellung zu überführen und andererseits das Siegel in die Schließstellung. Letztere ist dadurch gegeben, daß durch die erfolgte Spannung des Verschlusses dieser nicht mehr ohne übermäßige Kraftanstrengung geöffnet werden kann, sondern erst dann, wenn durch den mechanisch ohne Hitze- oder Druckeinwirkung nicht umkehrbaren Öffnungsvorgang das Siegel gelöst wird.

Die thermische Ausdehnung wird gemäß einem bevorzugten Ausführungsbeispiels mittels einer inkompressiblen Flüssigkeit mit großem Volumen-Ausdehnungskoeffizienten verwirklicht. Denkbar ist aber auch der Einsatz von Gasen oder Festkörpern, z.B. von Bimetallen oder Materialien mit hohem Ausdehnungskoeffizienten.

Bei Verwendung von Gasen ist eine vorzugsweise temperaturunabhängige, druckgesteuerte Ausführung möglich. Beispielsweise kann ein mit Normaldruck gefüllter Gassensor in Gestalt einer Kapsel bei Erreichen eines beliebigen Vakuums in der Vorvakuumphase oder der Trocknungsphase durch seine Expansion den Spann- bzw. bzw. Schaltvorgang bewirken.

Gemäß einem bevorzugten Ausführungsbeispiel ist die Verwendung einer Memory-Legierung (= Gedächtnis-Legierung) vorgesehen, die bei Überschreiten einer Umwandlungstemperatur von der martensitischen zur austenitischen Struktur übergeht, d.h. unterhalb der Umwandlungstemperatur hat eine aus dieser Legierung bestehende Feder keine oder nur eine sehr geringe Federrate. Oberhalb der Umwandlungstemperatur erhöht sich aufgrund der Veränderungen im Werkstoff die Federrate erheblich, so daß eine aus dieser Legierung bestehende Feder plötzlich eine erhöhte Vorspannung erhält. Nach der Erfindung ist eine solche Feder aus einer Memory-Legierung vorgesehen, die ein Verriegelungsorgan beim Überschreiten einer vorbestimmten Schalttemperatur in die Verriegelungsstellung überführt, in der das Verriegelungsorgan verrastbar ist, so daß es nach Unterschreiten der Temperatur nur durch manuelles Eindrücken wieder rückführbar ist, wobei in der gedrückten Öffnungsstellung wiederum eine Verrastung erfolgt. Wenn sich das Verriegelungsorgan unter der Wirkung der gespannten Memory-Feder in die Schließstellung bewegt hat ist ersichtlich, daß eine Sterilisation stattgefunden hat.

Weitere Ausgestaltungen der Erfindung ergeben sich aus den Unteransprüchen.

Nachstehend werden Ausführungsbeispiele der Erfindung anhand der Zeichnung beschrieben. In der Zeichnung zeigen:
Fig. 1 eine teilweise geschnittene Ansicht eines nach Art eines Vorhängeschlosses ausgebildeten Sicherungssiegels;
Fig. 2 einen Schnitt nach der Linie II-II gemäß Fig. 1;
Fig. 3 eine Schnittansicht eines an einer Behälterstirnwand angebrachten, einen Teil des Behälterverschlusses bildenden Sicherungssiegels mit Spannvorrichtung;
Fig. 4 einen Schnitt nach der Linie IV-IV gemäß Fig. 3.
Fig. 5 eine Stirnansicht eines Sterilisierbehälters mit einem durch ein Bimetall gesteuerten Sicherungssiegel-Verschluß.
Fig. 6 in größerem Maßstab einen Vertikalschnitt des Sicherungssiegel-Verschlusses gemäß Fig. 5.
Fig. 7 eine Stirnansicht eines Behälters mit Memory-Metall-Siegel;
Fig. 8 eine Seitenansicht des Behälterverschlusses nach Fig. 7;
Fig. 9 in größerem Maßstab den Ausschnitt X gemäß Fig. 7 mit offenem Sicherungssiegel;
Fig. 10 in größerem Maßstab den Ausschnitt X gemäß Fig. 7 in Schließstellung des Sicherungssiegels.

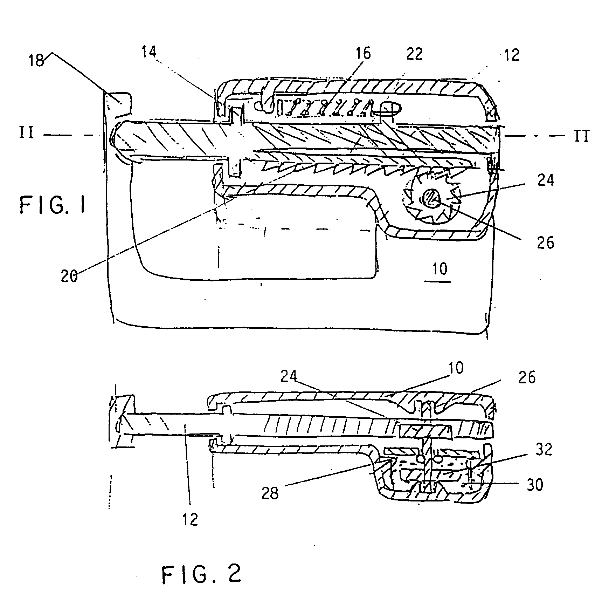

Das Sicherungssiegel gemäß Fig. 1 und 2 ist als loses Einzelteil in Form eines Vorhängeschlosses ausgebildet und es kann zur Versiegelung von Sterilisierbehältern verschiedenster Bauart benutzt werden. In entsprechender Abwandlung kann ein solches 'Vorhängeschloß' jedoch auch fest in eines der Verschlußteile von Deckel oder Behälterunterteil eingebaut werden.

Das Sicherungssiegel gemäß Fig. 1 und 2 weist ein Schloßgehäuse (10) mit einem linear verschiebbaren Schloßriegel (12) auf, der einen Anschlagflansch (14) besitzt, mit dem er gegen das Gehäuse beim Überführen in die Schließstellung anschlägt. Der Schloßriegel (14) ist durch eine Zugschraubenfeder (16) in die Schließstellung vorgespannt, und diese Schließfeder (16) ist in der aus Fig. 1 ersichtlichen Weise an einem Gehäuseansatz bzw. einem Ansatz des Riegels verankert.

Das Vorderende des Schloßriegels (12) wirkt mit einer an einem Gabelarm angebrachten Riegelfalle (18) zusammen, wenn sich das Sicherungssiegel in der aus Fig. 1 ersichtlichen Schließstellung befindet.

Der axial im Schloßgehäuse (10) geführte Schloßriegel (12) trägt eine Zahnstange (20), welche radial gegenüber dem Schloßriegel federnd ausgebildet ist. Nach dem Ausführungsbeispiel geschieht dies dadurch, daß der Schloßriegel axial mit einem Schlitz (22) versehen ist, jedoch kann die radiale Federung auch auf andere Weise zustande kommen. Diese Zahnstange weist Sägezähne auf, die mit den entsprechenden Zähnen eines Klinkenrades (24) zusammenwirken. Das Klinkenrad sitzt auf einer quer zum Schloßriegel im Schloßgehäuse drehbar gelagerten Welle (26), die in eine Kammer (28) einsteht und dort ein Rückhalterad (32) trägt. Die Kammer ist mit einer Legierung (30), insbesondere einer eutektischen Legierung angefüllt, die bei Raumtemperatur fest ist und bei Erreichen der Sterilisationstemperatur schmilzt.

Das Sicherungssiegel gemäß Fig. 1 und 2 wirkt wie folgt:
Aus der Stellung gemäß Fig. 1 und 2 kann bei erstarrtem Eutektikum (30) der Schloßriegel (12) manuell nach rechts verschoben werden, bis das vordere Ende des Schloßriegels an der Gehäusewand liegt und so den Eintritt zwischen Riegelfalle (18) und Gehäuse freigibt. Bei der mechanischen Rückführung des Schloßriegels kann die Zahnstange radial nach innen elastisch ausweichen und über das Klinkenrad (24) gleiten, welches durch das Eutektikum (30) drehfest im Gehäuse gehaltert ist. In diesem offenen Zustand wird das Sicherungssiegel am Behälterverschluß angebracht und der Behälter wird in den Sterilisator eingebracht. Bei Erreichen der Sterilisationstemperatur schmilzt das Eutektikum (30) und daher kann die Zahnstange (20) mit dem Schloßriegel (12) unter der Wirkung der Feder (16) ablaufen, da das nunmehr frei drehbare Klinkenrad (24) durch die Zahnstange in Drehung versetzt werden kann. Während der Sterilisierphase läuft der Schloßriegel in die aus Fig. 1 und 2 ersichtliche Stellung ab und nach Abkühlung erstarrt das Eutektikum und in dieser geschlossenen Stellung zeigt das Siegel an, daß seit einer Temperatur-(Druck)-einwirkung (vorzugsweise Sterilisiertemperatur, Sterilisationsdruck oder Trocknungsvakuum) der Behälterdeckel nicht vom Behälterunterteil abgehoben worden ist.

Das Sicherungssiegel gemäß Fig. 1 und 2 ist als loser Bestandteil in Form eines Vorhängeschlosses ausgebildet. Das Schloßgehäuse (10) kann aber auch fest an der Behälterstirnwand derart angebracht werden, daß der Schloßriegel (12) im Verschlußzustand vor der Verschlußlasche des Deckeloberteils zu liegen kommt oder in eine Ausnehmung desselben eingreift, so daß der Verschluß nicht geöffnet werden kann, ohne den Schloßriegel zurückzuführen, der dann erst wieder durch entsprechende Temperatureinwirkung in die Schließstellung überführbar ist.

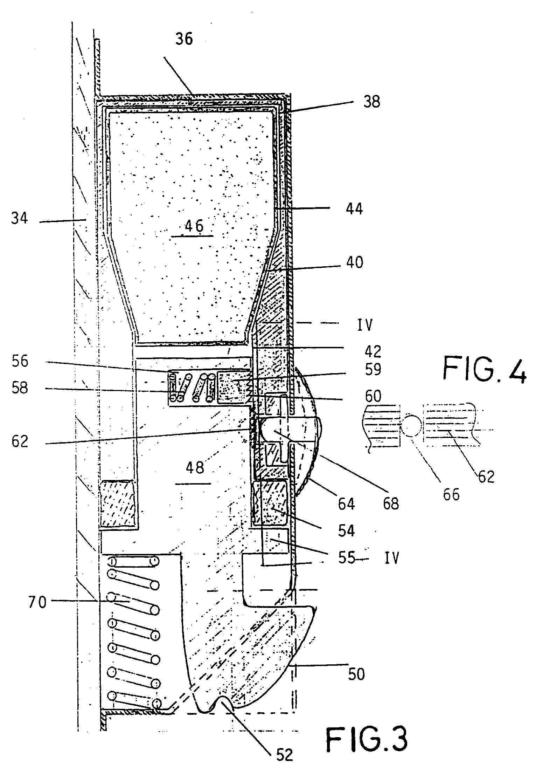

Das Ausführungsbeispiel nach Fig.3 und 4 stellt eine Erweiterung des Versiegelungsgedankens dar, wobei eine zusätzliche Funktion zustande kommt, nämlich das Anziehen des Deckels auf den Behälterunterteil unter Pressung der Dichtung. Die übrigen Funktionen (Plombierung und Prozeßindikator) bleiben erhalten.

An der Stirnseite des Behälterunterteils (34) ist ein Gehäuse (36) festgelegt, welches den unteren Teil des Deckelverschlusses bildet. Das Gehäuse bildet im oberen Teil eine Kammer (38), die sich nach unten in einem Abschnitt (40) verjüngt und in einem im Durchmesser verkleinerten Abschnitt (42) fortsetzt. In die Kammer ist ein dehnbarer Sack (44) eingesetzt, der mit einer inkompressiblen Flüssigkeit (46) gefüllt ist. In dem sich nach unten fortsetzenden Kammerteil (42) läuft axial geführt ein Behälterverschlußriegel (48), der im unteren aus dem Gehäuse vorstehenden Teils mit einer Auflaufschräge (50) für den am Deckel befestigten Verschlußteil versehen ist. Außerdem ist eine Ausnehmung (52) vorgesehen, in den der Verschlußteil des Deckels einrastet. Ein elastischer Ring (54) befindet sich zwischen dem Gehäuse (36) und einem Ringflansch (55) des Verschlußriegels. In einer radialen Sackbohrung (56) des Verschlußriegels befindet sich eine Druckschraubenfeder (58), die einen Block (59) abstützt, der an seiner nach außen weisenden Stirnfläche Sperrzähne (60) aufweist. Diese Sperrzähne wirken mit gehäusefesten Sperrzähnen (62) zusammen. Durch die gehäusefesten Sperrzähne (62) ist ein Schlitz (68) oder ein Loch geführt, durch das ein Stift (66) eines Auslösers (64) hindurchtreten kann, um die Sperrzähne (60) gegen den Druck der Feder (58) zurückzuschieben und die Verklinkung aufzuheben. Eine Druckschraubenfeder (70) stützt sich zwischen dem unteren Ende des Gehäuses und dem Flansch (55) ab und sucht den Verschlußriegel (48) nach oben zu schieben.

Die Funktion ist bei diesem Sicherungssiegel gemäß Fig. 3 und 4 die folgende:
Das Sicherungssiegel befindet sich in der Stellung gemäß Fig. 3 in Öffnungsstellung und die Verschlußlasche des Deckels kann nach Aufsetzen desselben über die Auflaufschräge (50) in die Ausnehmung (52) eingerastet werden. Wenn nun der Sterilisierbehälter im Sterilisator der Sterilisiertemperatur ausgesezt wird, dann dehnt sich die Flüssigkeit (46) innerhalb der Kammer aus. Sie kann zusammen mit dem Sack (44) jedoch nur nach unten ausweichen und drückt dabei auf die Stirnseite des Behälterverschlußriegels (48), so daß dieser entgegen der Wirkung der Feder (70) nach unten gepreßt wird und die Sperrzähne (60 und 62) in Eingriff kommen. Der Verschlußriegel (48) zieht über die Deckelverschlußlasche den Deckel auf den Behälter, so daß die Dichtung zusammengequetscht wird. Bei Abfall der Temperatur nimmt das Volumen der Flüssigkeit (46) ab, jedoch bleibt der Verschlußriegel (48) in seiner Stellung, weil er durch die ineinandergreifenden Sperrzähne (60 und 62) an einem Aufwärtshub gehindert ist. Der Behälter ist in dieser Stellung nicht ohne Gewalt zu öffnen und dadurch wird angezeigt, daß der Behälter seit der Sterilisation nicht geöffnet wurde. Das Sicherungssiegel wird dadurch gebrochen, daß auf den Auslöseknopf (64) gedrückt wird, dessen Stift (66) durch den Schlitz oder das Loch (68) auf die Sperrzähne (60) wirkt und damit diese und den Block (59) entgegen der Wirkung der Feder (58) zurückschiebt. Nunmehr kann der Verschlußriegel unter der Wirkung der Feder (70) nach oben ablaufen und wird dabei elastisch durch den Ring (54) abgefangen.

Bei dem nachstehend beschriebenen Ausführungsbeispiel ergibt sich bei der Temperaturerhöhung im Sterilisator ein Hub von 3mm:
- Volumen im Sensor ca. 1,50 ccm Öl mit 1,0 10 C Volumenausdehnungskoeffizient
- Trichterförmige Querschnittverengung auf 5x10 mm beim Riegel
- Bei Delta T 100 Grad : 10% Ausdehnung
- Verschiebung = 0,15 ccm/ 0,5 qcm = 0,3 cm = 3 mm

Selbstverständlich können weitere, beliebige Hubbewegungen realisiert werden durch Variation
- des Ausdehnungskoeffizienten der Flüssigkeit
- des Volumens der Flüssigkeit
- des Querschnitts des Riegels.

Bei entsprechender Bemessung kann auch die Ausdehnung eines Festkörpers oder eines Gases zur Verschiebung des Riegels herangezogen werden. Im Falle eines Gasvolumens kann dabei die Druckänderung als Steuerkriterium herangezogen werden, wobei die höhere Temperatur während der Sterilisation unterstützend wirkt.

Bei dem Ausführungsbeispiel nach Fig. 3 und 4 wird der Verschlußriegel (48) durch die Sperrzähne (60 und 62) in Verschlußstellung gehalten. Anstelle der Sperrzähne könnten auch andere Sperrmittel Verwendung finden. Beispielsweise könnte ein durch eine Blattfeder nach innen vorgespannter, im kalten Zustand am Verschlußriegel (48) anliegender Stift nach der Erwärmung und Verschiebung des Stiftes in ein dann vom Riegel freigegebenes Langloch des Verschlußkäfigs eingreifen, wodurch die Rückbewegung des Riegels nach Abkühlung unmöglich gemacht ist. Erst das manuelle Herausziehen des Stiftes gibt das Langloch wieder frei und läßt damit den Riegel in die Öffnungsstellung springen.

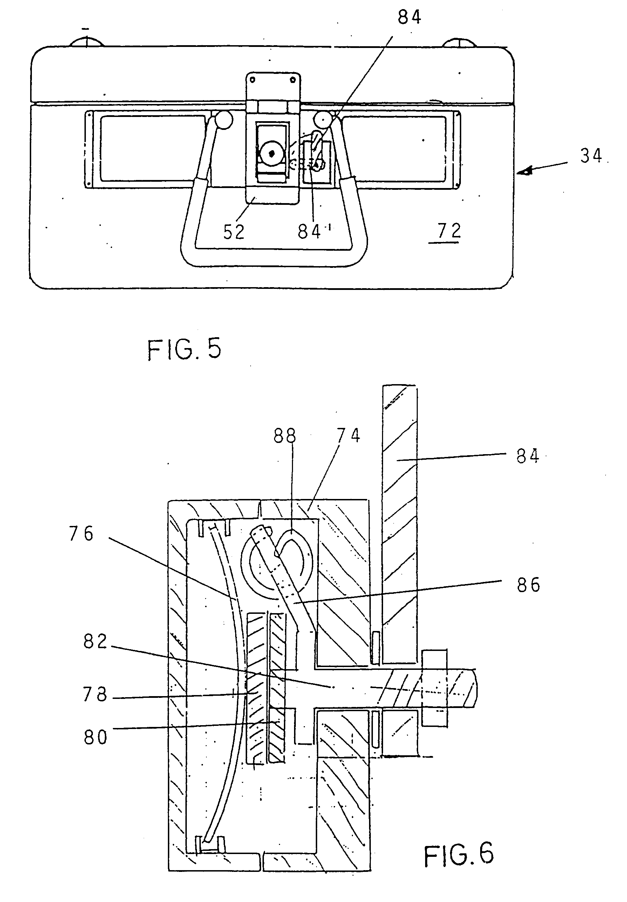

Ein weiteres Ausführungsbeispiel ist in den Figuren 5 und 6 dargestellt. Hier ist an der Stirnseite (72) des Behälterunterteils (34) ein Schloßgehäuse (74) in der Nähe der Deckelverschlußlasche (52) fixiert. In dem Gehäuse (74) ist ein Bimetall (76) in Gestalt eines Bimetallstreifens oder einer Schnappscheibe drehfest derart angeordnet, daß es sich in der Schnittebene gemäß Fig. 6 in Richtung von rechts nach links bzw. umgekehrt, bewegen kann. Die in Fig. 6 dargestellte Stellung ist die Stellung, die das Bimetall bei Raumtemperatur annimmt. Das Bimetall (76) trägt eine Zahnscheibe (78) mit axial gerichteten Sägezähnen, die mit den Sägezähnen einer weiteren Zahnscheibe (80) zusammenwirken. Die Zahnscheibe (80) sitzt auf einer im Gehäuse drehbar gelagerten Welle (82), die außerhalb des Gehäuses (74) einen Verschlußriegel (84) trägt, der in der Stellung gemäß Fig. 6 und der ausgezogenen Stellung gemäß Fig. 5 neben der Verschlußlasche (52) liegt und in der in Fig. 5 strichliert dargestellten Stellung (84′) vor der Verschlußlasche (52) zu liegen kommt und eine Öffnung derselben verhindert.

Die Welle (82) trägt innerhalb des Gehäuses (74) einen Arm (86), der durch eine gehäusefest abgestützte Druckschraubenfeder (88) in Schließrichtung des Verschlußriegels (84) vorgespannt ist. Die Verzahnung der Zahnscheiben (78, 80) ist so angeordnet, daß die Welle (82) in Eingriffsstellung der Zahnscheiben an einem Ablauf unter der Wirkung der Feder (88) gehindert ist. Aus der Schließstellung (84′) kann der Verschlußriegel jedoch durch Hand in die Öffnungsstellung gemäß Fig. 6 überführt werden, wobei die Zähne der Zahnscheiben (78 und 80) aufeinander ablaufen.

Unter der Wirkung der Sterilisiertemperatur weicht das Bimetall (76) gemäß Fig. 6 nach links aus, so daß die Verzahnungen der Zahnscheiben (78 und 80) ausgehoben werden und die Zahnscheibe (80) mit der Welle (82) und dem Verschlußriegel (84) unter der Wirkung der Schraubenfeder (88) in die Schließstellung (84′) ablaufen kann, in der die Verschlußlasche (52) des Behälterdeckels gesichert ist. Der Sterilisierbehälter wird in der in Fig. 6 dargestellten Öffnungsstellung des Verschlußriegels in den Sterilisator eingebracht und beim Erreichen einer vorbestimmten Temperatur, auf die das Bimetall (76) eingestellt ist, erfolgt eine Entkupplung und der Verschlußriegel geht in die Schließstellung über, die anzeigt, daß seit dem Sterilisiervorgang der Deckel des Behälters nicht mehr geöffnet worden ist.

Die in Fig. 6 dargestellte Schloßausbildung könnte auch als 'Vorhängeschloß' nach Art des Sicherungssiegels gemäß Fig. 1 und 2 ausgebildet sein, wobei der Verschlußriegel nicht durch eine axiale Linearbewegung in die Schließstellung überführt wird, sondern durch eine Schwenkbewegung.

Die vorbeschriebenen Ausführungsbeispiele beruhen in ihrer Schließwirkung auf einer Temperaturauslösung. Es soll jedoch auch vorbehalten bleiben, die Steuerung zusätzlich oder stattdessen durch Druckänderung vorzunehmen, d.h. durch Druckänderungen, die im Sterilisator vorhanden sind. So könnte beispielsweise das Bimetall (76) gemäß Fig. 6 durch eine Druckkapsel ersetzt werden, die beim Ansteigen des Drucks im Sterilisator während der Sterilisierphase zusammenbricht und den Verschlußriegel (84) nach Entkupplung in die Schließstellung ablaufen läßt. Kurz vor Beendigung des Sterilisierzyklus kann dann die Druckkapsel in der Entlüftungsphase wieder in ihren Ausgangszustand zurückkehren, in dem die Zahnscheiben eingekuppelt sind.

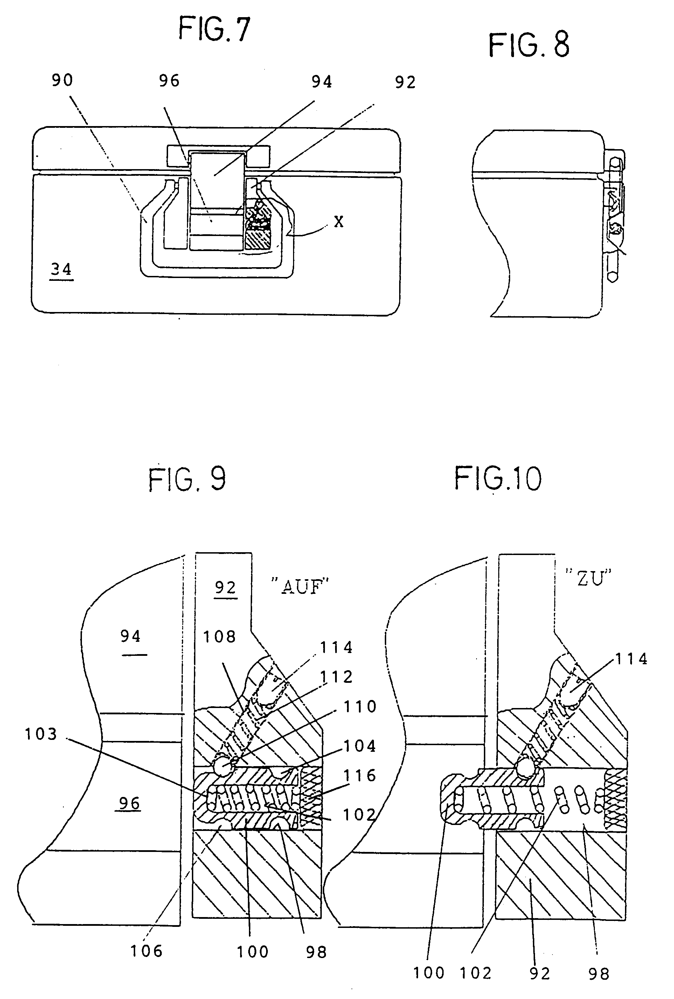

Ein bevorzugtes Ausführungsbeispiel ist in den Figuren 7 bis 10 dargestellt. Fig. 7 zeigt eine Stirnansicht eines Sterilisierbehälters, an dessen Unterteil ein Verschlußblock 92 angenietet ist, der den Behältergriff (90) schwenkbar lagert. Mit dem Verschlußblock 92 des Behälterunterteils (34) ist eine Verschlußlasche 94 in an sich bekannter Weise verspannbar. Diese Verschlußlasche 94 weist im unteren Teil eine Vertiefung 96 auf. Der Verschlußblock 92 weist im Bereich dieser Vertiefung 96 eine Durchgangsbohrung 98 auf, in der ein hohler Druckknopf 100 verschiebbar gelagert ist. Die Bohrung 98 ist an der Außenseite durch ein Verschlußstück 116 abgeschlossen, das in ein Gewinde der Bohrung eingeschraubt werden kann oder in die Bohrung eingesteckt und durch eine Madenschraube gesichert ist. An dem Verschlußstück 116 stützt sich das eine Ende einer Druckschraubenfeder 102 ab, die von dem hohlen Druckknopf 100 umschlossen ist und sich mit ihrem anderen Ende am Boden 103 des Druckknopfes abstützt. Die Feder 102 besteht aus einer Memory-Metall-Legierung, die unterhalb einer Schalttemperatur praktisch keine Federrate aufweist. Bei Erwärmung über den Umwandlungspunkt erhöht sich aufgrund von Veränderungen innerhalb der Legierung die Federrate erheblich. D.h. die Feder 102 schiebt den Druckknopf 100 gemäß Fig. 9 nach links in die aus Fig. 10 ersichtliche Verriegelungsstellung, in der der Druckknopf im Bereich der Vertiefung 96 der Lasche 94 liegt, so daß diese Lasche nicht aufgeklappt und der Behälterdeckel daher nicht abgenommen werden kann.

Die Umwandlungstemperatur kann beispielsweise bei 65 ° liegen. Sie kann aber auch höher gewählt werden, muß jedoch unterhalb der Sterilisiertemperatur liegen.

Der Druckknopf wird sowohl in der aus Fig. 9 ersichtlichen Öffnungsstellung als auch in der aus Fig. 10 ersichtlichen Schließstellung verrastet. Zu diesem Zweck weist der Druckknopf 100 auf seinem äußeren Umfang eine hintere Ringnut 104 und eine vordere Ringnut 106 auf. Der Verschlußblock 92 weist eine die Bohrung 98 treffende schrägverlaufende Bohrung 108 auf, in die eine Rastkugel 110 eingesetzt ist, die durch eine Rastkraftfeder 112 in die Rastnut 104 bzw. 106 eingedrückt wird. Die Rastkraftfeder 112, die als Druckschraubenfeder ausgebildet ist, stützt sich mit ihrem hinteren Ende an einer Stellschraube 114 ab, die in einen Gewindeabschnitt der Bohrung 108 eingeschraubt ist. Die aus den Figuren 7 bis 10 ersichtlichen Bauteile sind auch auf der gegenüberliegenden Stirnseite des Behälters angeordnet, und zwar zweckmäßigerweise jeweils auf der gleichen Seite der Verschlußlasche 94, d.h. z.B. jeweils auf der gemäß Fig. 7 rechten Seite dieser Verschlußlasche. Dadurch wird gewährleistet, daß der Behälterdeckel im verriegelten Zustand nicht angehoben werden kann und durch die gleiche Lageanordnung wird erreicht, daß der Druckknopf mit dem gleichen Finger der linken bzw. rechten Hand durch Eindrücken in gleicher Richtung von der Verriegelungsstellung in die Öffnungsstellung überführt werden kann.

Die aus Fig. 7 bis 10 ersichtliche bevorzugte Ausführungsform arbeitet wie folgt:

Der Sterilisierbehälter wird mit geöffnetem Sicherungssiegel gemäß Fig. 7 und 9 in den Sterilisator eingebracht. Sobald die Umwandlungstemperatur der Memory-Metall-Legierung der Feder 102 erreicht ist, erhöht sich die Federrate der Feder 102 erheblich, d.h. sie drückt den Druckknopf 100 aus der Stellung gemäß Fig. 9 nach links in die Stellung gemäß Fig. 10. Dabei wird die Rastkugel 110 aus der Rastnut 106 ausgehoben und fällt dann in Verriegelungsstellung in die Rast 104 ein. In dieser Stellung verbleibt der Druckknopf 100 auch dann, wenn die Temperatur unter die Umwandlungstemperatur fällt und die Feder 102 praktisch keine Federkraft mehr ausübt. Der Druckknopf 100 verhindert in dieser Stellung gemäß Fig. 10 ein Öffnen der Verschlußlaschen 94 an beiden Enden und läßt erkennen, daß einerseits der Behälter einer Sterilisation unterworfen ist und andererseits eine Öffnung des Behälters nach der Sterilisation noch nicht stattgefunden hat. Zur Benutzung werden die Druckknöpfe 100 auf beiden Seiten des Behälters in die Bohrung 98 zurückgedrückt, bis die Rastkugel 110 wieder in die Rastnut 106 einrastet. Dann können die Verschlußlaschen 94 verschwenkt und der Deckel geöffnet werden. Nachdem der Druckknopf einmal in die Stellung gemäß Fig. 9 eingedrückt ist, besteht keine Möglichkeit, diesen in die Verriegelungsstellung zu überführen, außer wenn er der Temperatur im Sterilisator ausgesetzt wird.

Vorstehend wurden nur einige Ausführungsbeispiele beschrieben, die das Wesen der Erfindung veranschaulichen sollen. Das erfindungsgemäße Prinzip der Verriegelung durch ein Sicherungssiegel kann aber noch auf vielerlei andere Weise verwirklicht werden. So kann beispielsweise ein mechanisch aufspannbarer Verschluß Anwendung finden, dessen Schließbewegung durch Einsetzen oder Anbringen eines durch die Sterilisation sich verändernden Organs bewirkt wird. Dieses Organ kann ein thermisch schrumpfendes, oder durch Druckeinwirkung platzendes, oder ein sich durch Dampfeinwirkung auflösendes Teil sein, welches während der Sterilisation den Verschluß herstellt und vor erneutem Sterilisiervorgang ersetzt wird. Ferner kann beispielsweise die auf Temperatur ansprechende Memory-Metall-Legierungs-Feder 102 gemäß Fig. 7 bis 10 durch eine Druckkapsel ersetzt werden, die sich während des Sterilisiervorganges aufbläht und den Druckknopf 100 gemäß Fig. 9 nach links verschiebt.

## Patentansprüche

1. Sicherungssiegel für Sterilisierbehälter mit einer definierten Schließstellung, welche gewährleistet, daß der Sterilisierbehälter seit seiner Sterilisation geschlossen gehalten wurde und mit einer Öffnungsstellung, die auf eine zwischenzeitliche Öffnung hinweist,
gekennzeichnet durch die folgenden Merkmale:
- ein mechanisches Verriegelungsorgan (12;48;84;100) weist eine definierte Schließstellung und eine definierte Öffnungsstellung auf;
- das Verriegelungsorgan verbindet in Schließstellung Behälter und Behälterdeckel derart, daß ein Öffnen des Deckels verhindert ist;
- das Verriegelungsorgan gibt in Öffnungsstellung den Behälterdeckel frei;
- das Verriegelungsorgan ist in die Schließstellung vorgespannt;
- das Verriegelungsorgan ist bei Umgebungstemperatur manuell aus der Schließstellung in die Öffnungsstellung überführbar;
- das Verriegelungsorgan ist in der Öffnungsstellung gegen Auslösung durch ein Temperaturglied gesperrt, welches bei Erreichen einer vorbestimmten Temperatur das Verriegelungsorgan freigibt und unter der Wirkung der Vorspannung in die Schließstellung ablaufen läßt.

2. Sicherungssiegel für Sterilisierbehälter mit einer definierten Schließstellung, welche gewährleistet, daß der Sterilisierbehälter seit seiner Sterilisation geschlossen gehalten wurde und mit einer Öffnungsstellung, die auf eine zwischenzeitliche Öffnung hinweist,
gekennzeichnet durch die folgenden Merkmale:
- ein mechanisches Verriegelungsorgan weist eine definierte Schließstellung und eine definierte Öffnungsstellung auf;
- das Verriegelungsorgan verbindet in Schließstellung Behälter und Behälterdeckel derart, daß ein Öffnen des Deckels verhindert ist;
- das Verriegelungsorgan gibt in Öffnungsstellung den Behälterdeckel frei;
- das Verriegelungsorgan ist bei Umgebungsdruck manuell aus der Schließstellung in die Öffnungsstellung überführbar;
- das Verriegelungsorgan ist in der Öffnungsstellung gegen Auslösung durch ein Druckglied gesperrt, welches bei Erreichen eines vorbestimmten Druckes das Verriegelungsorgan freigibt und in die Schließstellung ablaufen läßt.

3. Sicherungssiegel nach Anspruch 1,
dadurch gekennzeichnet, daß das Verriegelungsorgan (100) durch eine Feder (102) aus einer Memory-Metall-Legierung, die erst nach Überschreiten der Memory-Umwandlungstemperatur ihre Spannkraft erhält, in die Schließstellung überführt wird.

4. Sicherungssiegel nach Anspruch 3,
dadurch gekennzeichnet, daß das Verriegelungsorgan ein Druckknopf (100) ist, der in Schließstellung und in Öffnungsstellung verrastbar (104, 106) ist, und der willkürlich manuell in die Öffnungsstellung überführbar ist.

5. Sicherungssiegel nach Anspruch 2,
dadurch gekennzeichnet, daß das Verriegelungsorgan durch eine expandierfähige Druckkapsel bei im Sterilisierbehälter herrschenden Vakuum in die Schließstellung überführbar ist.

6. Sicherungssiegel nach Anspruch 1,
dadurch gekennzeichnet, daß es einen Verschlußriegel (12) aufweist, der durch eine Feder (16) in die Schließstellung vorgespannt und durch einen Temperatursensor (30) oder einen Drucksensor in der Öffnungsstellung gehalten ist, und der über eine Einrichtungskupplung (20, 24) in die Öffnungsstellung überführbar ist.

7. Sicherungssiegel nach den Ansprüchen 1 und 6,
dadurch gekennzeichnet, daß der Verschlußriegel (12) einen Sperrklinken-Mechanismus (20) aufweist, dessen Sperrklinke (24) durch eine bei Schalttemperatur schmelzende Legierung (30) so lange in Öffnungsstellung gehalten ist, bis die Schalttemperatur erreicht ist.

8. Sicherungssiegel nach Anspruch 1,
dadurch gekennzeichnet, daß das Siegel über den Verschluß des Sterilisierbehälters, dessen Deckel auf seinen Unterteil (34) unter Herstellung eines Dichtungsdruckes auf die Behälterdichtung drückt während es in den Schließzustand überführt wird, und daß als Temperatursensor ein Ausdehnungsorgan (56) vorgesehen ist, welches den Behälterverschluß (48, 52) beim Übergang in den Schließzustand spannt und verriegelt.

9. Sicherungssiegel nach Anspruch 8,
dadurch gekennzeichnet, daß das Ausdehnungsorgan auf einen Verschlußriegel (48) des Behälterverschlusses entgegen Federkraft (70) einwirkt, und daß dieser Verschlußriegel (48) durch einen Verklinkungsmechanismus (60, 62) in Schließstellung gehalten wird.

10. Sicherungssiegel nach Anspruch 1,
dadurch gekennzeichnet, daß am Behälterunterteil in der Nähe der Verschlußlasche (52) des Deckels ein Schloßgehäuse (74) fixiert ist, das den Steuermechanismus für einen linear beweglichen oder schwenkbaren Verschlußriegel beherbergt, der in Schließstellung vor der Verschlußlasche (52) des Deckels liegt und deren Öffnung verhindert.

11. Sicherungssiegel nach Anspruch 10,
dadurch gekennzeichnet, daß der schwenkbare Verschlußriegel (84) von einer drehbar im Gehäuse (74) gelagerten Welle (82) getragen wird, die unter Federvorspannung steht und mit einer Rutschkupplung (78, 80) zusammenwirkt, die durch Temperatur und/oder Druckunterschiede während der Sterilisation entkuppelbar ist.

## Claims

1. Safety seal for sterilisation containers having a defined closed position assuring that the sterilisation container remained closed after sterilisation thereof, and an open position indicating that the container has been opened before,
characterised by the following features:
- a mechanical locking member (12; 48; 84; 100) comprises a defined closed position and a defined opened position;
- the locking member is operative to join, in the closed position, the container with the container lid such that opening of the lid is prevented;
- the locking member, in its open position, releases the container lid;
- the locking member is biased into its closed position;
- the locking member is manually transferable from the closed position into the open position at ambient temperature;
- the locking member, in its open position, is blocked against unlocking by a temperature dependant means releasing such that the locking member, at a predetermined temperature, is movable by means of said bias into the closed position.

2. Safety seal for sterilisation containers having a defined closed position assuring that the sterilisation container remained closed after sterilisation thereof, and an open position disclosing unsealing of the container in the meantime,
characterised by the following features:
- a mechanical locking member (12; 48; 84; 100) comprises a defined locking position and a defined opening position;
- the locking member is effective to connect in its locking position the container with the container lid so that opening of the lid is prevented;
- the locking member is biased into its locking position;
- the locking member when under ambient temperature can be moved from the locking position into the opening position;
- the locking member in its open position is confined against release by pressure dependant means releasing at a predetermined pressure the locking member to move into the closed position.

3. Safety seal in accordance with claim 1, characterised in that the locking member (100) is moved into the closed position by a spring made of a memory-metal-alloy producing its spring force only on exceeding the memory conversion temperature.

4. Safety seal in accordance with claim 3, characterised in that the locking member comprises a push button (100) lockable in closed position and open position (104, 106) and which is movable by hand into its open position.

5. Safety seal in accordance with claim 2, characterised in that the locking member is movable into the closed position by an expandable pressure capsule when subjected to the vacuum within the sterilising container.

6. Safety seal in accordance with claim 1, characterised in that it comprises a locking bar (12) biased into the closed position by a spring (16) and retained in its open position by a temperature sensor (30) or by a pressure sensor being moveable into the open position by a one-way clutch (20, 24).

7. Safety seal in accordance with claims 1 and 6, characterised in that the locking bar (12) comprises a ratchet mechanism (20) the pawl (24) thereof being retained by an alloy melting at a predetermined melting temperature in its open position until this melting temperature is exceeded.

8. Safety seal in accordance with claim 1, characterised in that the seal is operative for pressing the lid onto the lower part of the container by means of the container closure member, when it is moved into its closed position and that the temperature sensor comprises an expanding member (46) tensioning and locking the closure members (48, 52) of the container during movement into the closed position.

9. Safety seal in accordance with claim 8, characterised in that the expending member engages a closure bolt (48) of the container closure member against spring force (70) and that the closure bolt (48) is retained in its closed position by a latching-in device (60, 62).

10. Safety seal in accordance with claim 1, characterised in that, adjacent to the locking flap (52) of the lid, a lock housing (74) is provided housing the control mechanism for a linearly or pivotably movable locking bar, said locking bar, in closed position, engaging the locking flap (52) of the lid and preventing opening thereof.

11. Safety seal in accordance with claim 10, characterised in that the pivotably moving locking bar (84) is supported by a shaft (82) rotatably mounted within the housing (74), said shaft being spring biased and cooperating with a slip clutch (78, 80) which is disengagable by temperature and/or pressure differences during sterilisation.

## Revendications

1. Dispositif de sécurité pour une cuve de stérilisation comportant une position de fermeture définie qui garantit que la cuve de stérilisation a été maintenue fermée depuis la dernière stérilisation et une position d'ouverture qui indique que la cuve à été ouverte entretemps, caractérisé par les éléments suivants:
- un organe de verrouillage (12; 48; 84; 100) mécanique présente une position de fermeture définie et une position d'ouverture définie;
- en position de fermeture, l'organe de verrouillage relie la cuve et le couvercle de la cuve de telle sorte qu'une ouverture du couvercle est impossible;
- en position d'ouverture, l'organe de verrouillage libère le couvercle de la cuve;
- en position de fermeture, l'organe de verrouillage est précontraint;
- à la température ambiante, l'organe de verrouillage peut être amené manuellement de la position de fermeture dans la position d'ouverture;
- en position d'ouverture, un élément réagissant à la température bloque le déclenchement de l'organe de verrouillage, lequel élément réagissant à la température, lorsqu'une température prédéterminée est atteinte, libère l'organe de verrouillage et laisse celui-ci revenir dans sa position de fermeture sous l'action de la précontrainte.

2. Dispositif de sécurité pour une cuve de stérilisation comportant une position de fermeture définie qui garantit que la cuve de stérilisation a été maintenue fermée depuis la dernière stérilisation et une position d'ouverture qui indique que la cuve à été ouverte entretemps, caractérisé par les éléments suivants:
- un organe de verrouillage mécanique présente une position de fermeture définie et une position d'ouverture définie;
- en position de fermeture, l'organe de verrouillage relie la cuve et le couvercle de la cuve de telle sorte qu'une ouverture du couvercle est impossible;
- en position d'ouverture, l'organe de verrouillage libère le couvercle de la cuve;
- à la pression ambiante, l'organe de verrouillage peut être amené manuellement de la position de fermeture dans la position d'ouverture;
- en position d'ouverture, un élément réagissant à la pression bloque le déclenchement de l'organe de verrouillage, lequel élément réagissant à la pression, lorsqu'une pression prédéterminée est atteinte, libère l'organe de verrouillage et laisse celui-ci revenir dans sa position de fermeture.

3. Dispositif de sécurité selon la revendication 1, caractérisé par le fait que l'organe de verrouillage (100) est amené dans la position de fermeture par un ressort (102) en alliage métallique à mémoire de forme qui ne reçoit sa force de rappel qu'à partir du moment où la température de transition est dépassée.

4. Dispositif de sécurité selon la revendication 3, caractérisé par le fait que l'organe de verrouillage est un doigt de pression (100) qui s'encliquette dans la position de fermeture et dans la position d'ouverture (104, 106) et qui peut être amené manuellement de façon délibérée dans la position d'ouverture.

5. Dispositif de sécurité selon la revendication 2, caractérisé par le fait que l'organe de verrouillage est amené dans la position de fermeture par une capsule manométrique expansible, lorsque le vide règne à l'intérieur de la cuve de stérilisation.

6. Dispositif de sécurité selon la revendication 1, caractérisé par le fait qu'il comporte un verrou (12) qui, en position de fermeture, est précontraint par un ressort (16) et en position d'ouverture, est maintenu par un capteur de température (30) ou un capteur de pression et peut être amené en position d'ouverture par un dispositif d'accouplement (20, 24).

7. Dispositif de sécurité selon les revendications 1 et 6, caractérisé par le fait que le verrou (12) comporte un mécanisme à cliquet (20) dont le cliquet (24) est retenu dans la position d'ouverture par un alliage (30) qui fond à la température de déclenchement, jusqu'au moment où la température de déclenchement et atteinte.

8. Dispositif de sécurité selon la revendication 1, caractérisé par le fait que, lorsqu'on l'amène en position de fermeture, l'élément de sécurité, par l'intermédiaire du dispositif de fermeture de la cuve de stérilisation, presse le couvercle sur la partie inférieure (34) de la cuve en exerçant une pression d'étanchéité sur le joint de la cuve et par le fait qu'il est prévu comme capteur de température un organe (56) expansible qui, lors du passage en position de fermeture, tend le dispositif de fermeture et le verrouille.

9. Dispositif de sécurité selon la revendication 8, caractérisé par le fait que l'organe expansible agit sur verrou (48) du dispositif de fermeture de la cuve à l'encontre de la force du ressort (70) et par le fait que ce verrou (48) est maintenu en position de fermeture par un mécanisme à encliquetage (60, 62).

10. Dispositif de sécurité selon la revendication 1, caractérisé par le fait qu'un boîtier de verrou (74) est fixé sur la partie inférieure de la cuve à proximité de l'attache de fermeture (52) du couvercle, lequel boîtier contient le mécanisme de commande pour un verrou à déplacement linéaire ou pivotant qui, en position de fermeture est placé devant l'attache de fermeture (32) du couvercle et empêche l'ouverture de celui-ci.

11. Dispositif de sécurité selon la revendication 10, caractérisé par le fait que le verrou (84) pivotant est supporté par un axe (82) qui est monté tournant dans le boîtier (74), est sollicité par ressort et coopère avec un accouplement à friction (78, 80) qui peut être débrayé par des gradients de température et/ou de pression lors de la stérilisation.
